# EUROPEAN PATENT SPECIFICATION

(11) **EP 2 164 555 B1**
(45) Date of publication and mention of the grant of the patent: **26.03.2014**
(21) Application number: 08776505.3
(22) Date of filing: 25.06.2008
(51) Int. Cl.: A61F 2/95, A61M 25/01

(54) **DEVICE FOR CONTROLLING A CATHETER**
VORRICHTUNG ZUR STEUERUNG EINES KATHETERS
DISPOSITIF PERMETTANT DE COMMANDER UN CATHÉTER

(30) Priority: 25.06.2007 FR 0704541
(43) Date of publication of application: 24.03.2010
(73) Proprietor: Stentys S.A.S., 75002 Paris (FR)
(72) Inventor: ATLANI, David, F-95280 Jouilly Le Moutier (FR); ISSENMANN, Gonzague, F-92110 Clichy (FR)
(74) Representative: Jeannet, Olivier
(86) International application number: PCT/IB2008/052546
(87) International publication number: WO 2009/001309

(56) References cited:
- EP-A- 1 046 406
- WO-A-98/23241
- US-A1- 2004 006 380
- US-A1- 2005 060 016
- US-A1- 2007 060 999

## Description

The present invention relates to a device for controlling a catheter used for deploying a stent.

It is known how to re-establish the diameter of a body lumen by placing in this lumen a radially expandable tubular frame currently called a "stent", that may or not be self-expanding. For its implantation, a self-expandable stent is placed on an elongated support forming the axial core of a catheter and is maintained in a condition of radial contraction by a sheath that covers the stent, this sheath being slidably engaged on the support. The sheath may be slid relative to the support so as to release the stent.

The stent should be deployed at a specific location of the body lumen, particularly if it has to be implanted at a bifurcation or when it is used for implanting a heart valve.

In order to achieve deployment of the stent, the practitioner should implement a "fixed point", i.e. maintain with one hand, a portion of the catheter fixed, connected to said support, and displace with his/her other hand another portion of the catheter connected to said sheath.

This standard, relatively empirical technique is not always very easy to apply and does not exclude a risk of inaccuracy as regards the positioning of the stent relatively to the body lumen upon deploying this stent.

Document US 2005/060016 describes a handle of a catheter for delivering a stent, which comprises a thumb wheel and a rack-and-pinion system in order to move back a restraining sheath of the stent, and a locking system providing immobilization of the rack. The thumb wheel is located so as to be found at the level of the thumb of the user when the handle is grasped. This handle may be grasped both by a right hand and a left hand.

The handle according to this prior document does not find a remedy to the aforementioned drawbacks.

An object of the present invention is to find a remedy to these drawbacks, by providing a control device that allows deployment of the stent in a specific location of the body lumen, and this in such a way that a user may easily apply it.

This device may be used for the deployment of a self-expanding stent. The device may comprise a catheter for conveying and deploying the stent, equipped with structure for supporting the stent and with a deployment mechanism for deploying the stent, the device further comprising a portion connected to said stent-supporting structure and a portion connected to said stent-deploying mechanism, said portion connected to the stent-supporting structure being in the form of a handle which may be grasped by a hand of the user.

According to embodiments of the invention, the handle comprises at least two front bosses allowing it to be supported on a relatively stable surface, such as the operating table, the height of this bosses being such that this support may be achieved in spite of the presence of the fingers of the user around the handle.

With the handle according to embodiments of the invention, it is thereby possible to achieve the "fixed point" under the best conditions, while obtaining immobilization of this handle.

The handle comprises two front bosses, i.e. located at the end of this handle connected to the catheter, positioned transversely, i.e. substantially perpendicularly to the longitudinal axis of the handle.

Stability of the handle when it pivots according to this longitudinal axis is also obtained.

Preferably, the handle has a symmetrical shape relatively to a longitudinal median plane, so that it may be equally grasped by a right hand or a left hand of a user.

The handle may therefore be grasped in an undifferentiated way by a left or right hand depending on the side of the patient selected for approaching the implantation site.

Preferably,
- the handle inwardly comprises a longitudinal guiding mechanism that comprises a rack, and a longitudinal aperture opening out into the area of the handle that is located opposite to the thumb of the user when the handle is grasped by the hand of this user;
- the device comprises a portion connected to said stent-deploying mechanism, as a mobile slider along said guiding mechanism, this slider including a thumb wheel pivotably mounted thereon, connected to a pinion engaged with said rack, and an actuation button, this thumb wheel and this button protruding through said longitudinal aperture,
said rack being laid out in a location of the guiding mechanism such that said pinion is engaged with this rack on a first part of the actuation of said stent-deploying mechanism in the deployment direction of this stent, which substantially corresponds to a deployment of the stent, which still remains insufficient for immobilizing this stent relative to the body lumen, and such that said pinion is disengaged from this rack on a second part of the actuation of said stent-deploying mechanism in the deployment direction of this stent, which substantially corresponds to a deployment of this stent, sufficient for immobilizing this stent relative to the body lumen or at the very least limiting the mobility of this stent relative to this body lumen.

The device according to embodiments of the invention thus comprises a handle which may be firmly grasped by a hand of the user, the thumb of this hand will face said thumb wheel and said button once this grasping is performed; the thumb wheel may be actuated in rotation by the thumb of the user, in order to perform a slow and controlled backward movement of the slider on said first actuation part; this rotation therefore achieves a slow and controlled gradual deployment of the stent, while the handle properly remains in the hand of the user thanks to the actuation of this thumb wheel by the thumb, so as to ensure that the implantation location of the stent may be perfectly retained. Further rotation of the thumb wheel results in said pinion coming out of engagement with the rack; the button may then be used for performing faster actuation of said stent-deploying mechanism, and therefore fast deployment of the stent; this disengagement is however only performed once the stent has been sufficiently deployed in order to have a certain hold on the body lumen so as to be longitudinally immobilized relatively to this body lumen, or at the very least to have become slightly mobile relatively to the latter.

During these operations, the other hand of the user, freed by using the device according to embodiments of the invention, may be placed at the entry point of the catheter into the catheter guide or introducer and may allow, if necessary, fast correction of the positioning of the catheter.

The practitioner however keeps the option of acting either on the thumb wheel or directly on the actuation button, according to his/her preference; in this second case, by the fact that the thumb is engaged with the rack on said first part of the actuation of said deployment mechanism, a certain control of the deployment of the stent may be retained.

Preferably, the device may comprise an anti-rotation mechanism for preventing rotation of the thumb wheel in the opposite direction to the one allowing actuation of said stent-deploying mechanism in the direction of this deployment, or at the very least limiting free rotation of this thumb wheel in this opposite direction.

With the anti-rotation mechanism, it is possible to prevent or limit the elastic return of the stent-deploying mechanism in the direction opposite to the direction of deployment.

The anti-rotation mechanism may for example comprise friction against the thumb wheel and the slider and/or the rack, or a system of notches and pawl(s), or a system of bosses/cavities laid out on the adjacent faces of the thumb wheel and the slider and/or the rack, such that the passing of the bosses along each other during the rotation of the thumb wheel forms "hard points" which have to be crossed in order to allow this rotation.

Embodiments of the invention will be better understood, and other features and advantages of the latter will become apparent, with reference to the appended schematic drawings, illustrating as a non-limiting example, preferred embodiments of the relevant control device.
Fig. 1 is a perspective view of a device according to embodiments of the invention;
Fig. 2 is an exploded perspective view of the device of Fig. 1;
Fig. 3 is a view of it similar to Fig. 1 after the device is grasped by the hand of a user;
Fig. 4 is a median longitudinal sectional view of it, in a position of a device configured to include a slider, and
Fig. 5 is a view of a device, similar to Fig. 4, configured to include a slider.

The figures illustrate a device 1 for controlling a catheter used for deploying a stent.

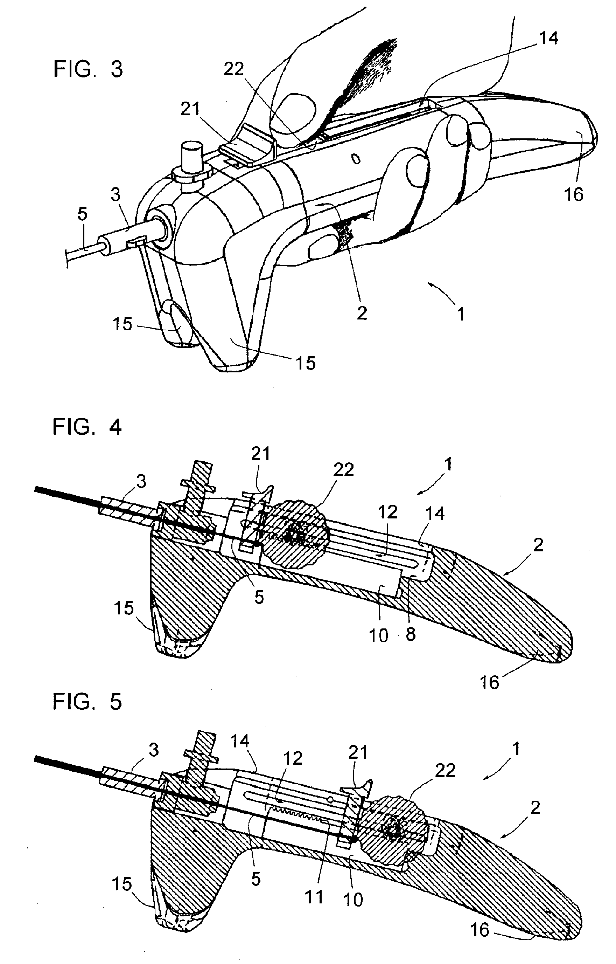

The catheter (not shown) comprises an axial core forming a support for receiving the stent and a sliding sheath which, in a sliding position, covers the stent in order to keep it in a condition of radial contraction and which, in another sliding position, releases the deployment of this stent. The device 1 comprises a shell forming a handle 2, connected to said axial core through a protruding rod 3 which it comprises, and a slider 4 connected to a cable 5 itself connected to said sheath. The handle 2 may be grasped by a hand of a user, as shown in Fig. 3.

In the description hereafter, the terms of "proximal" and "distal" are taken into consideration relative to the point connecting the handle 2 to the catheter, "proximal" designating an area closer to this connection point and "distal" an area further away from this same point.

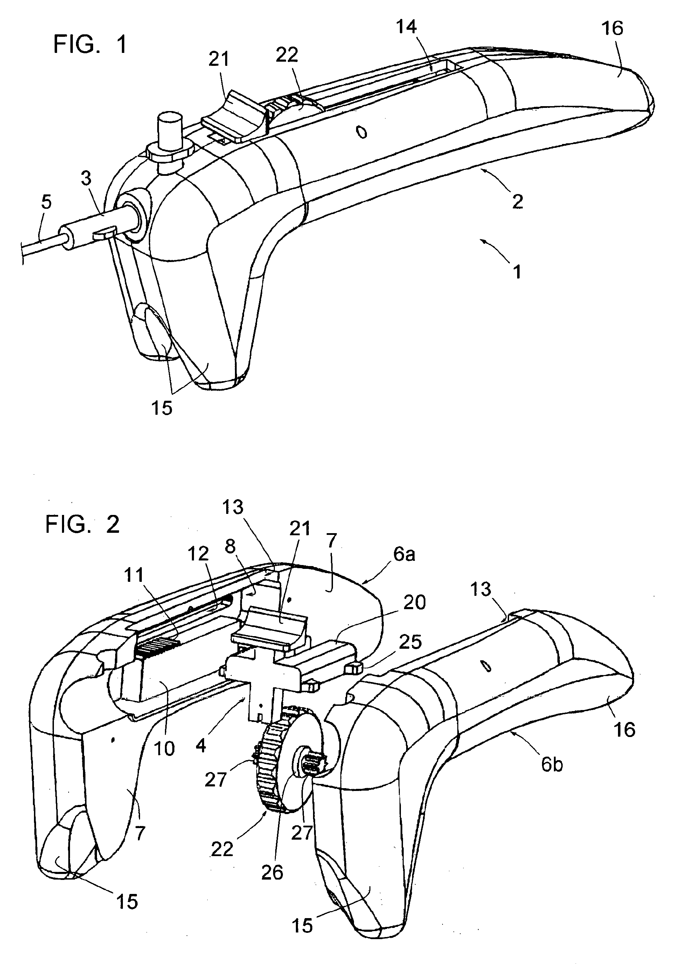

As this is more particularly apparent in Fig. 2, the handle 2 may be formed by two assembled half-shells 6a, 6b, which comprise planar surfaces 7 at their longitudinal end portions, so that they may come against each other in the position of assembly, and median recesses 8 for receiving the slider 4. These half-shells 6a, 6b, are preferably symmetrical relative to their assembly plane defined by the surfaces 7, this plane corresponding to the median longitudinal plane of the handle 2.

Each half-shell 6a, 6b comprises:
- a block 10 laid out in the recess 8 on the front portion of the upper face of which a rack 11 is laid out;
- a longitudinal groove 12 laid out sideways in the half-shell 6a, 6b along said upper face of the block 10; and
- a longitudinal notch 13 laid out opposite this same upper face of the block 10, in the edge of the half-shell 6a, 6b; both notches 13 of both half-shells 6a, 6b thereby form a longitudinal aperture 14 which opens out into the area of the handle 2 located facing the thumb of the user when this handle 2 is grasped by the hand of this user.

Each half-shell 6a, 6b further forms a proximal boss 15 and comprises a slightly curved rear end 16. The height of each boss 15 is larger than the thickness of the fingers of a hand, as this is shown in Fig. 3, so that both transversely positioned bosses 15 which the handle 2 comprises after assembly, and half-shells 6a, 6b may be supported against a stable surface, for example, an operating table, without this support being an obstacle to the engagement of the fingers of the user other than the thumb around the handle 2.

The slider 4 preferably has a U-shaped body 20, including a median button 21 and receiving a thumb wheel 22.

The body 20 on its opposite longitudinal edges, includes protruding slides 25 that are able to be engaged and slideable in the grooves 12.

The median button 21 is connected to the body 20 by a portion able to be engaged and to slide in the aperture 14. It has a concave front shape adapted to the area supporting the thumb or index finger of the user.

The thumb wheel 22 is intended to be mounted in the space existing between both branches of the U which the body 20 forms. It comprises two axial pivots 26 intended to be received in housings which the body 20 comprises, forming bearings for receiving these pivots 26. The latter are axially extended by pinions 27 which will engage with both racks 11 when the slider 4 is placed between both half-shells 6a, 6b and when these half-shells are assembled together.

The slider 4 is thus mobile relative to the handle 2 between a front position shown in Fig. 4, corresponding to the position for covering the stent with the sheath, and a rear position shown in Fig. 5, corresponding to the position for having the stent completely released by the sheath.

As apparent in Figs. 2, 4 and 5, both racks 11 may only occupy a front portion of the length of the recesses 8. Their lengths may be such that the pinions 27 are engaged with them on a first portion of the sliding course of the sheath, substantially corresponding to a deployment of the stent, remaining still insufficient for immobilizing this stent relatively to the body lumen into which the catheter is engaged, and that the pinions 27 are disengaged from them on a second portion of the sliding course of the sheath, substantially corresponding to a deployment of the stent, sufficient for immobilizing this stent relatively to the body lumen.

In practice, the catheter is introduced into the body lumen until its stent-supporting portion is positioned at the implantation site. The handle 2 is then grasped by a hand of the user and placed on a stable surface, for example, the operating table. This grasping is preferably accomplished in the way shown in Fig. 3, in which four fingers of the hand may be engaged around the handle 2 and the thumb arriving at the level of the thumb wheel 22 and the button 21. The other hand of the user may be placed at the entry point of the catheter into the catheter guide and may if necessary allow fast correction of the positioning of the catheter.

The thumb wheel 22 is then actuated into rotation by the thumb of the user, in order to perform a slow and controlled backward movement of the slider 4 on said first portion of the sliding course. This rotation therefore achieves a slow and controlled gradual deployment of the stent, while the handle 2 remains properly held in the hand, which ensures that the implantation location of the stent may be retained. Further rotation of the thumb wheel 22 results in the pinions 27 disengaging from the racks 11; the button 21 may then be used for performing faster sliding of the sheath, and therefore faster deployment of the stent. This disengagement however may be performed only once that the stent has been sufficiently deployed in order to have a certain hold on the body lumen so as to be longitudinally immobilized relatively to this body lumen, or at the very least to have become slightly mobile relatively to the latter.

The practitioner keeps the option of acting either on the thumb wheel 22 or directly on the button 21 to perform a backward movement of the sheath; in this second case, by the fact that the thumb wheel 22 is engaged with the racks 11 on said first part of the sliding course, it is possible to retain a certain control on the deployment of the stent.

Embodiments of the invention thus provide a device for controlling a catheter used for deploying a stent having, as compared with the homologous devices of the prior art, the decisive advantage of being perfectly adapted for allowing deployment of the stent in a specific location of the body lumen, and this in such a way that a user may easily apply it.

## Claims

1. Device (1) for controlling a catheter used for deploying a stent, the catheter being equipped with structure for supporting the stent and with a deployment mechanism for deploying this stent, the device (1) comprising a portion (2) connectable to said stent-supporting structure and a portion (4) connectable to said deployment mechanism, said portion connectable to the stent-supporting structure being in the form of a handle (2) which may be grasped by a hand of the user, and the handle (2) comprises two front bosses (15), i.e. located at an end of this handle (2) connected to the catheter, positioned transversely, i.e. substantially perpendicularly to a longitudinal axis of the handle (2), the height of each boss (15) being larger than the thickness of the fingers of a user's hand, so that the bosses (15) allow the handle (2) to be supported on a relatively stable surface, such as an operating table, such that this support may be achieved in spite of the presence of fingers of the user around the handle (2).

2. Device (1) according to claim 1, **characterized in that** the handle (2) has a symmetrical shape relatively to a longitudinal median plane, so that it may be equally grasped by a right hand or a left hand of a user.

3. Device (1) according to claim 1 or claim 2, **characterized in that**:
- the handle inwardly comprises a longitudinal guiding mechanism (12) that comprises a rack, and a longitudinal aperture (14) opening out into the area of the handle (2) located opposite to a thumb of the user when the handle (2) is grasped by the hand of this user; and **in that**
- said portion connected to said stent-deploying mechanism comprises a mobile slider (4) along said guiding mechanism (12), which includes a thumb wheel (22) pivotably mounted thereon, connected to a pinion (27) engaged with said rack (11), and an actuation button (21) protruding through said longitudinal aperture (14),
said rack (11) being laid out in a location of the guiding mechanism (12) such that said pinion (27) is engaged with this rack (11) on a first part of the actuation of said stent-deploying mechanism in a deployment direction of this stent, which substantially corresponds to a deployment of the stent, which still remains insufficient for immobilizing this stent relative to a body lumen, and such that said pinion (27) is disengaged from this rack (11) on a second part of the actuation of said stent-deploying mechanism in the deployment direction of this stent, which substantially corresponds to a deployment of this stent, sufficient for immobilizing this stent relative to the body lumen or at the very least limiting the mobility of this stent relative to this body lumen.

4. Device (1) according to any of claims 1 to 3, **characterized in that** the handle (2) is formed by two assembled half-shells (6a, 6b).

5. Device (1) according to claim 4, **characterized in that** the two half-shells (6a, 6b) are symmetrical relatively to an assembly plane, this plane corresponding to the median longitudinal plane of the handle (2).

6. Device (1) according to claim 4 or 5, **characterized in that** each half-shell (6a, 6b) comprises:
- a block (10) laid out in a median recess (8) on a front portion of an upper face of which a rack (11) is laid out, and
- the thumb wheel (22) comprises two pinions (27) engaging with both racks (11) of both half-shells (6a, 6b) when these half-shells are assembled together.

7. Device (1) according to any of claims 4 to 6, **characterized in that** each half-shell (6a, 6b) comprises a longitudinal groove (12), both grooves (12) of both half-shells (6a, 6b) forming said longitudinal guiding mechanism.

8. Device (1) according to any of claims 4 to 7, **characterized in that** each half-shell (6a, 6b) comprises a longitudinal notch (13), both notches (13) of both half-shells (6a, 6b) forming said longitudinal aperture.

9. Device (1) according to any of claims 1 to 8, **characterized in that** it comprises an anti-rotation mechanism for preventing rotation of the thumb wheel in the opposite direction to the one allowing actuation of said stent-deploying mechanism in the direction of this deployment, or at the very least limiting free rotation of this thumb wheel in this opposite direction, this anti-rotation mechanism comprising a system of notches and pawls, or a system of bosses/cavities laid out on the adjacent faces of the thumb wheel and the slider and/or the rack, such that the passing of the bosses along each other during the rotation of the thumb wheel forms "hard points" which have to be crossed in order to allow this rotation.

## Patentansprüche

1. Vorrichtung (1) zur Steuerung eines Katheters, der zum Einsetzen eines Stents verwendet wird, wobei der Katheter mit einer Struktur zum Stützen des Stents und einem Einsetzmechanismus zum Einsetzen des Stents ausgestattet ist, wobei die Vorrichtung (1) einen Abschnitt (2), der mit der Stent stützenden Struktur verbindbar ist, und einen Abschnitt (4), der mit dem Einsetzmechanismus verbindbar ist, umfasst, wobei der Abschnitt, der mit der Stent stützenden Struktur verbindbar ist, in Form eines Griffs (2) vorliegt, der mit einer Hand von dem Benutzer gegriffen werden kann, und wobei der Griff (2) zwei vordere Vorsprünge (15) umfasst, die sich an einem Ende des Griffs (2) befinden, der mit dem Katheter verbunden ist, quer positioniert, d.h. im Wesentlichen senkrecht zu einer Längsachse des Griffs (2), wobei die Höhe jedes Vorsprungs (15) größer ist als die Dicke der Finger der Hand eines Benutzers, sodass die Vorsprünge (15) ein Abstützen des Griffs (2) auf einer relativ standfesten Oberfläche, wie einem Operationstisch, erlauben, sodass das Abstützen trotz der Anwesenheit der Finger des Benutzers um den Griff (2) erreicht werden kann.

2. Vorrichtung (1) nach Anspruch 1, **dadurch gekennzeichnet, dass** der Griff (2) eine symmetrische Form in Bezug auf eine in Längsrichtung verlaufende Medianebene aufweist, sodass er gleichermaßen von einer rechten Hand oder einer linken Hand des Benutzers gegriffen werden kann.

3. Vorrichtung (1) nach Anspruch 1 oder Anspruch 2, **dadurch gekennzeichnet, dass**:
- der Griff innen einen in Längsrichtung verlaufenden Führungsmechanismus (12) umfasst, der eine Zahnstange und eine in Längsrichtung verlaufende Öffnung (14) umfasst, die sich nach außen in den Bereich des Griffs (2) öffnet, der sich gegenüber einem Daumen des Benutzers befindet, wenn der Griff (2) von der Hand den Benutzers gegriffen wird; und dass
- der Abschnitt, der mit dem Stent einsetzenden Mechanismus verbunden ist, einen beweglichen Schieber (4) entlang des Führungsmechanismus (12) umfasst, der ein darauf drehbar angebrachtes Daumenrad (22), das mit einem Ritzel (27) verbunden ist, das mit der Zahnstange(11) in Eingriff ist, und einen Betätigungsknopf (21) umfasst, der aus der in Längsrichtung verlaufenden Öffnung (14) hervorsteht,
wobei die Zahnstange (11) an einer Stelle des Führungsmechanismus (12) angeordnet ist, sodass das Ritzel (27) mit der Zahnstange (11) bei einem ersten Teil der Betätigung des Stent einsetzenden Mechanismus in einer Einsetzrichtung des Stent in Eingriff ist, die im Wesentlichen einem Einsetzen des Stents entspricht, die noch unzureichend bleibt, um den Stent in Bezug auf ein Körperlumen unbeweglich zu machen, und sodass das Ritzel (27) bei einem zweiten Teil der Betätigung des Stent einsetzenden Mechanismus in der Einsetzrichtung des Stents, die im Wesentlichen dem Einsetzen des Stents entspricht, aus dem Eingriff mit der Zahnstange (11) gelöst wird, was ausreicht, um den Stent in Bezug auf das Körperlumen unbeweglich zu machen oder zumindest die Beweglichkeit des Stents in Bezug auf das Körperlumen zu begrenzen.

4. Vorrichtung (1) nach einem der Ansprüche 1 bis 3, **dadurch gekennzeichnet, dass** der Griff (2) aus zwei zusammengesetzten Halbschalen (6a, 6b) gebildet ist.

5. Vorrichtung (1) nach Anspruch 4, **dadurch gekennzeichnet, dass** die zwei Halbschalen (6a, 6b) in Bezug auf eine Montageebene symmetrisch sind, wobei die Montageebene der in Längsrichtung verlaufenden Medianebene des Griffs (2) entspricht.

6. Vorrichtung (1) nach Anspruch 4 oder 5, **dadurch gekennzeichnet, dass** jede Halbschale (6a, 6b), umfasst:
- einen Block (10), der in einer medianen Aussparung (8) angeordnet ist, wobei auf einem vorderen Abschnitt einer oberen Seite des Blocks eine Zahnstange (11) angeordnet ist, und
- das Daumenrad (22) zwei Ritzel (27) umfasst, die mit beiden Zahnstangen (11) beider Halbschalen (6a, 6b) in Eingriff sind, wenn die Halbschalen zusammengesetzt sind.

7. Vorrichtung (1) nach einem der Ansprüche 4 bis 6, **dadurch gekennzeichnet, dass** jede Halbschale (6a, 6b) eine in Längsrichtung verlaufende Nut (12) umfasst, wobei beide Nuten (12) beider Halbschalen (6a, 6b) den in Längsrichtung verlaufenden Führungsmechanismus bilden.

8. Vorrichtung (1) nach einem der Ansprüche 4 bis 7, **dadurch gekennzeichnet, dass** jede Halbschale (6a, 6b) eine in Längsrichtung verlaufende Einkerbung (13) umfasst, wobei beide Einkerbungen (13) beider Halbschalen (6a, 6b) die in Längsrichtung verlaufende Öffnung bilden.

9. Vorrichtung (1) nach einem der Ansprüche 1 bis 8, **dadurch gekennzeichnet, dass** sie einen Anti-Dreh-Mechanismus zum Verhindern einer Drehung des Daumenrads in entgegengesetzter Richtung zu der Richtung, die ein Betätigen des Stent einsetzenden Mechanismus in Richtung des Einsetzens erlaubt, oder zumindest zum Begrenzen einer freien Drehung des Daumenrads in die entgegengesetzte Richtung umfasst, wobei der Anti-Dreh-Mechanismus ein System aus Einkerbungen und Sperrklinken, oder ein System aus Vorsprüngen/Hohlräumen umfasst, das an den aneinander angrenzenden Flächen des Daumenrads und des Schiebers und/oder der Zahnstange angeordnet ist, sodass das Passieren der Vorsprünge aneinander entlang während der Drehung des Daumenrads "harte Punkte" bildet, die überwunden werden müssen, um die Drehung zu erlauben.

## Revendications

1. Dispositif (1) de commande d'un cathéter servant au déploiement d'un stent, le cathéter étant équipé d'une structure pour supporter le stent et d'un mécanisme de déploiement pour déployer ce stent, le dispositif (1) comprenant une partie (2) apte à être reliée à ladite structure de support du stent et une partie (4) apte à être reliée audit mécanisme de déploiement du stent, ladite partie reliée à la structure de support du stent étant sous la forme d'une poignée (2) apte à être saisie par une main de l'utilisateur, la poignée (2) comprenant deux bossages antérieurs (15), c'est-à-dire situés au niveau de l'extrémité de cette poignée (2) reliée au cathéter, disposés transversalement, c'est-à-dire perpendiculairement à l'axe longitudinal de la poignée (2), la hauteur de chaque bossage (15) étant plus grande que l'épaisseur des doigts de la main d'un utilisateur, de sorte que les bossages (15) permettent à la poignée (2) de prendre appui sur une surface relativement stable, telle qu'une table d'opération, de telle sorte que cette prise d'appui puisse se faire nonobstant la présence des doigts de l'utilisateur autour de la poignée (2).

2. Dispositif (1) de commande selon la revendication 1, **caractérisé en ce que** la poignée (2) présente une forme symétrique par rapport à un plan médian longitudinal, de telle sorte qu'elle peut être saisie indifféremment par une main droite ou une main gauche d'un utilisateur.

3. Dispositif (1) de commande selon la revendication 1 ou la revendication 2, **caractérisé en ce que** :
- la poignée comprend intérieurement un moyen (12) de guidage longitudinal, au niveau d'une partie duquel est aménagée une crémaillère (11), qui comprend une ouverture longitudinale (14) débouchant dans la zone de la poignée (2) se trouvant en regard du pouce de l'utilisateur lorsque la poignée (2) est saisie par la main de cet utilisateur ; et **en ce que**
- ladite partie reliée auxdits mécanisme de déploiement du stent est sous forme d'un coulisseau (4) mobile le long dudit moyen de guidage (12), qui comporte une molette (22) montée pivotante sur lui, reliée à un pignon (27) en prise avec ladite crémaillère (11), et un bouton d'actionnement (21), cette molette (22) et ce bouton d'actionnement (21) faisant saillie au travers de ladite ouverture longitudinale (14),
ladite crémaillère (11) étant aménagée en un emplacement du moyen de guidage (12) tel que ledit pignon (27) est en prise avec cette crémaillère (11) sur une première partie de l'actionnement desdits mécanisme de déploiement du stent dans le sens du déploiement de ce stent, qui correspond substantiellement à un déploiement du stent restant encore insuffisant pour immobiliser ce stent par rapport au conduit corporel, et que ledit pignon (27) est hors de prise d'avec cette crémaillère (11) sur une deuxième partie de l'actionnement desdits mécanisme de déploiement du stent dans le sens du déploiement de ce stent, qui correspond substantiellement à un déploiement du stent suffisant pour immobiliser ce stent par rapport au conduit corporel ou tout au moins limiter la mobilité de ce stent par rapport à ce conduit corporel.

4. Dispositif (1) de commande selon l'une des revendications 1 à 3, **caractérisé en ce que** la poignée (2) est formée par deux demi-coquilles (6a, 6b) assemblées.

5. Dispositif (1) de commande selon la revendication 4, **caractérisé en ce que** les demi-coquilles (6a, 6b) sont symétriques par rapport à leur plan d'assemblage, ce plan correspondant au plan longitudinal médian de la poignée (2).

6. Dispositif (1) de commande selon la revendication 4 ou la revendication 5, **caractérisé en ce que** chaque demi-coquille (6a, 6b) comprend :
- un bloc (10) aménagé dans un évidement médian (8), sur la partie antérieure de la face supérieure duquel est aménagée une crémaillère (11), et
- la molette (22) comprend deux pignons (27) venant en prise avec les deux crémaillères (11) des deux demi-coquilles (6a, 6b) lorsque ces demi-coquilles sont assemblées l'une à l'autre.

7. Dispositif (1) de commande selon l'une des revendications 4 à 6, **caractérisé en ce que** chaque demi-coquille (6a, 6b) comprend une rainure longitudinale (12), les deux rainures 12 des deux demi-coquilles (6a, 6b) formant ledit moyen de guidage longitudinal.

8. Dispositif (1) de commande selon l'une des revendications 4 à 7, **caractérisé en ce que** chaque demi-coquille (6a, 6b) comprend une échancrure longitudinale (13), les deux échancrures (13) des deux demi-coquilles (6a, 6b) formant ladite ouverture longitudinale (14).

9. Dispositif (1) de commande selon l'une des revendications 1 à 8, **caractérisé en ce qu'**il comprend des moyens anti-rotation empêchant la rotation de la molette dans le sens inverse de celui permettant d'actionner lesdits mécanisme de déploiement du stent dans le sens de ce déploiement, ou tout au moins limitant la rotation libre de cette molette dans ce sens inverse.
